# FASCICULE DE BREVET EUROPEEN

(11) **EP 0 665 734 B1**
(45) Date de publication et mention de la délivrance du brevet: **21.08.1996**
(21) Numéro de dépôt: 93922867.2
(22) Date de dépôt: 14.10.1993
(51) Int. Cl.: A61F 7/00

(54) **COUSSIN THERMOTHERAPEUTIQUE**
THERMOTHERAPEUTISCHES KISSEN
THERMAL TREATMENT CUSHION

(30) Priorité: 20.10.1992 GB 9222037
(43) Date de publication de la demande: 09.08.1995
(73) Titulaire: GAUDREAULT, Yvon, Joliette, Québec J6E 1X7 (CA); LEBEAU, Monique, St-Charles Borromée, Québec J6E 1K5 (CA); ROBITAILLE, Rita, St-Charles Borromée, Québec J6E 7J8 (CA)
(72) Inventeur: GAUDREAULT, Yvon, Joliette, Québec J6E 1X7 (CA); LEBEAU, Monique, St-Charles Borromée, Québec J6E 1K5 (CA); ROBITAILLE, Rita, St-Charles Borromée, Québec J6E 7J8 (CA)
(74) Mandataire: Bonnetat, Christian
(86) Numéro de dépôt international: CA9300432
(87) Numéro de publication internationale: WO9408537

(56) Documents cités:
- WO-A-90/14059
- US-A- 2 393 446
- US-A- 4 865 012
- J. Microw. Power Electromagn. Energy (USA), 1987, Vol. 22, Nr. 1, Pages 35-39

## Description

### DOMAINE DE L'INVENTION

L'invention a trait à des dispositifs de coussins flexibles, permettant le dégagement continu de chaleur ou de froid, pour un temps donné, sur un membre du corps d'un mammifère (humain ou animal).

Un coussin thermothérapeutique comportant les caractéristiques du préambule de la revendication 1 est connu du document WO-A-90/14059.

### ÉTAT DE LA TECHNIQUE

Les dispositifs thermothérapeutiques connus de transfert de chaleur permettent de soulager la douleur, les courbatures, les affections psychogènes chez les mammifères (créatures homéothermes, tels que les humains), la résorption des régions de membres enflés (e.g., oedème), entre autres choses; ils peuvent également procurer un effet cosmétique en ouvrant les pores de la peau, comme dans les traitements de beauté et les applications dermatologiques. On y parvient grâce à l'accroissement de la circulation sanguine dans les membres sur lesquels est appliqué le dispositif de transfert de chaleur.

Les effets du transfert thermique localisé à des parties du corps avec ces dispositifs connus de transfert thermique, sont bien documentés dans le domaine. De tels dispositifs de transfert de chaleur comprennent notamment:
(a) un simple sac rempli d'eau chaude, ou à l'opposé, un enveloppement glacé rempli de glace d'eau;
(b) des couvertures dites "électriques", constituées de feuilles isolées flexibles à plusieurs épaisseurs, et une cellule thermique intégrée aux feuilles et dégageant une chaleur continue lorsque son fil électrique est branché à la prise électrique de secteur dans le mur de la maison;
(c) des enveloppes de vapeur de type chimique, qui dégagent de la chaleur lorsque l'enveloppe est déchirée, ce qui permet le déclenchement d'une réaction exothermique par oxydation de l'agent chimique réagissant à l'air, agent chimique qu'on retrouve dans l'enveloppe; ceci pour une période de temps d'environ une demi-heure - elles sont utilisées par exemple par les skieurs alpins à l'intérieur de leurs gants; voir par exemple le brevet américain No 3,874,504 délivré à M. Verakas;
(d) des coussins thermiques remplis de sels minéraux, ayant une enveloppe flexible enfermant du matériau minéral granulaire (habituellement du sable ou de l'ébonite); le coussin est pré-chauffé dans un four avant usage, afin de permettre une lente diffusion de la chaleur absorbée par les cristaux de minéraux - voir par exemple la demande de brevet internationale No PCT\EP90\00785 publiée le 29 novembre 1990 au nom de M. Herbert Hans WAGNER (WO-A-90/14059);
(e) des enveloppes thermiques à âme gélifiée, avec une enveloppe enfermant un matériau à l'état de gel ayant un contenu important en eau associé à une âme absorbant les liquides, cette âme comprenant par exemple du propylène glycol et de la formaldéhyde (afin d'accroître la gamme de températures de l'état de gel avant d'atteindre l'état de fusion ou de liquéfaction) - voir par exemple les brevets américains No 4,488,552 délivré le 18 décembre 1984 à la société *Micropak manufacturing, inc*., et No 4,920,964 concédé le 1er mai 1990 à la société *Jack Frost Laboratories inc*.; ces enveloppes thermiques dégageant lentement la chaleur accumulée, après avoir été soumises à un pré-chauffage pour un certain temps dans un fourneau, de la même façon que les coussins thermiques remplis de sels minéraux, supra .

Ces dispositifs de transfert de chaleur de l'état de la technique, même s'ils sont efficaces, comportent un certain nombre de désavantages. Pour ce qui est des couvertures électriques, des champs magnétiques sont créés. Actuellement, une controverse fait rage quant à savoir si oui ou non une exposition de longue durée de tissus vivants à de hauts niveaux de champs électromagnétiques, pourrait être associée au développement cellulaire cancéreux. De récentes études poussées sur les rapports existant entre les champs électriques et la matière vivante ont soulevé de vives inquiétudes chez les scientifiques, quant aux dangers pour la santé associés aux couvertures électriques. Voir par exemple l'ouvrage de référence "CRC Handbook of biological effects of electromagnetic fields", éditeur: Charles POLK, CRC Press, Boca Raton, Floride [1986].

Les enveloppes chimiques à vapeur sont du type jetable après usage, i.e. qu'elles ne peuvent pas être réutilisées. Leur coût est par conséquent élevé, par rapport à des coussins réutilisables de transfert de chaleur tels ceux remplis de sels minéraux ou pourvus d'une âme gélifiée. De plus, le dosage de l'agent actif à l'intérieur de l'enveloppe scellée est vital, de façon à ne pas surchauffer l'enveloppe - en effet, la presse a fait état il y a quelques années de quelques cas de blessures corporelles causées par l'exposition de la peau à de telles enveloppes chimiques à vapeur, provoquées vraisemblablement par une quantité mal mesurée (i.e. une quantité plus grande que requise) d'agent chimique actif dans l'enveloppe.

Les coussins thermiques de type gel ont tendance à crever si la période de préchauffage dépasse la période de temps recommandée. La marge d'erreur est très faible, comme on le reconnaît d'ailleurs candidement dans le mémoire descriptif des deux brevets américains d'enveloppe à gel, précités. Un coussin crevé devient inutilisable, and peut même constituer un danger. De plus, les enveloppes à gel ont tendance à produire une chaleur du type "humide", qui ne répond peut être pas aux besoins de tous.

Les coussins de transfert de chaleur à base de sels minéraux comportent d'autres désavantages. En raison du contenu extrêmement faible en eau qu'on retrouve dans le matériau minéral granulaire de l'enveloppe du coussin, il y a tendance à ce que se produise une réelle pulvérisation des sels minéraux au fur et à mesure que se répètent les mouvements de pliage du coussin flexible lorsque celui-ci vient épouser les contours des diverses parties des membres. Eventuellement, ces granules deviennent réduits à l'état de poudre (i.e. leur granulométrie décroît progressivement), jusqu'à ce qu'ils puissent s'échapper du coussin au travers les pores de la paroi de l'enveloppe. De plus, des matériaux minéraux comme du sable définissent des granules chacun ayant des arêtes externes relativement vives; de telles arêtes seront certainement inconfortables à la peau puisqu'elles viendront indirectement en contact avec celle-ci au travers la paroi de l'enveloppe du coussin. Également, parce que la granulométrie du sable est assez faible, il a été découvert par ces trois co-inventeurs que le déplacement du matériau granulaire tel qu'induit par la charge d'une pression, à l'intérieur de l'enveloppe de coussin, et par conséquent leur écoulement distributif, avait tendance à créer assez facilement des vides sans granule à l'intérieur de l'enveloppe de coussin. En d'autres mots, le matériau granulaire, plutôt que de se distribuer -comme il serait souhaitable - de façon bien répartie un peu partout à l'intérieur de l'enveloppe du coussin, ce sable aura plutôt tendance à se ramasser aux deux extrémités opposées du coussin, sous la simple influence des forces de gravitation sur les granules, alors que la partie intermédiaire du coussin (i.e. cette même partie de coussin qui vient justement en contact avec la partie du corps à être soulagée) manquera justement de matériau d'âme dégageant de la chaleur (les deux parties correspondantes des parois principales opposées de l'enveloppe viendront s'appuyer à plat l'une contre l'autre), ce qui constitue une inefficacité.

### BUTS DE L'INVENTION

Le but principal de l'invention est donc de prévoir un dispositif de transfert thermique, qui visera à répondre aux désavantages décrits dans le précédent paragraphe dit "d'état de la technique".

Plus particulièrement, un but important de cette invention est de prévoir un coussin de transfert de chaleur ou de froid, réalisé uniquement en des composantes "naturelles".

Un autre but important de l'invention est que le coussin de transfert de chaleur comprennent du matériau granulaire dont la granulométrie, le contenu en eau, et le volume de chargement de l'enveloppe, soient tels, qu'un écoulement bien réparti du matériau granulaire (i.e. une bonne conservation de la forme du coussin) à l'intérieur de l'enveloppe du coussin soit obtenu de façon répétée suite au pliage du coussin, sans qu'il ne se forme de vides sans grains à l'intérieur du coussin.

Un but d'ensemble de l'invention est de fournir un tel dispositif de transfert de chaleur, qui soit d'un faible coût de fabrication, de type réutilisable, de manipulation sécuritaire, et dont la méthode d'emploi soit simple et rapide.

Comme autres buts de l'invention, il y a: que le coussin soit résistant à l'éclatement par suite de surchauffe; que l'enveloppe du coussin soit réalisée en un matériau isolant thermiquement de sorte qu'une application directe sur la peau du coussin soit sécuritaire du point de vue santé; et que ce coussin thermothérapeutique ait aussi bien une inertie thermique élevée qu'une inertie d'humidité élevée.

### SOMMAIRE DE L'INVENTION

Compte tenu des buts de l'invention, l'on prévoit un coussin thermothérapeutique comportant les caractéristiques de la revendication 1.

Préférablement, ladite ligne de couture de la feuille en cotcn est orientée vers l'extérieur par rapport à ladite enceinte de l'enveloppe, de sorte que ladite enceinte de l'enveloppe définit une surface intérieure lisse telle qu'on favorise un libre écoulement équi-réparti des grains à l'intérieur du coussin, lorsque le coussin est plié.

Avantageusement, lesdits grains d'avoine définissent une granulométrie sensiblement constante, ladite granulométrie de grain étant spécifiquement choisie pour permettre un écoulement libre bien réparti des grains à l'intérieur de ladite enveloppe de coussin, même après de nombreux pliages du coussin, tout en empêchant clairement la formation de vides sans grains dans l'enceinte formée à l'intérieur de ladite enveloppe du coussin, aux portions coudées du coussin thermique plié; ladite granulométrie du grain demeurant sensiblement constante durant l'emploi du coussin, même après son pliage répété.

Il serait souhaitable que la densité de ladite enveloppe de coton soit approximativement de 203 grammes par mètre carré (203 g\m²) [six onces par verge carrée].

### DESCRIPTION DÉTAILLÉE DES RÉALISATIONS DE L'INVENTION

Le coussin flexible thermique pour le transfert thermique au membre d'une personne consiste en une enveloppe de tissu enfermant une masse de grains de céréales, préférablement de l'avoine. Le contenu en eau de l'avoine est préférablement contrôlé de façon précise pour demeurer entre 9 et 14 % par poids du poids total de l'avoine. La quantité de grains d'avoine à l'intérieur de l'enveloppe de coton, et la granulométrie du grain d'avoine, sont choisis avec soin de façon telle qu'est obtenu un écoulement distributif bien réparti de l'avoine à l'intérieur de l'enceinte formée par l'enveloppe de coton, après déformation, de sorte que la formation de vides sans grains localisés est sensiblement empêchée. Selon une réalisation de l'invention, un coussin avec un kilo de masse de remplissage d'avoine est préchauffé pendant une à deux minutes à l'intérieur d'un four à micro-ondes, avant emploi. L'inertie thermique de l'avoine permet une diffusion de chaleur pendant à peu près une demi-heure. Ce même coussin pourrait également être placé au congélateur pour quatre à six heures, afin de diffuser par la suite du froid à une partie oedémateuse d'un membre.

L'avoine utilisé dans le coussin est préparé tel qu'il suit. En premier lieu, l'avoine est entreposé à l'intérieur d'un silo à grains conventionnel, où son humidité naturelle ou son contenu en eau deviendra ajusté à à peu près 13 à 16 % par poids. A ce niveau de contenu d'eau, l'inertie d'humidité est relativement faible, i.e. que les grains d'avoine répondront rapidement à une variation de contenu d'eau lorsque les niveaux d'humidité dans l'air ambiant varient. Les grains d'avoine sont d'abord ébarbés, pour éliminer les contaminants biologiques, puis criblés, pour enlever les contaminants inorganiques, de sorte que les grains d'avoine seront susceptibles de maintenir leurs caractéristiques naturelles pour une longue période de temps. Les grains d'avoine sont alors séchés à l'intérieur d'un réservoir de séchage, avec du gaz propane, afin de réduire leur contenu en eau à au moins 13 ou 14 % en poids; préférablement, il y aura un séchage plus poussé, afin d'amener le contenu d'eau à un niveau encore plus bas, jusqu'à 12, et préférablement jusqu'à 11, et même jusqu'à aussi bas qu'environ 9 % en poids; cependant, les co-inventeurs ont découverts que le niveau idéal était d'environ 10,5 % de contenu en eau. Pour y arriver, il faudra prévoir un certain nombre de jours de chauffage constant.

Sous 10 % en poids de contenu d'eau d'avoine, le coussin thermique devient rapidement très chaud, ce qui peut constituer un danger pour se brûler la peau. Sous 9 % en poids de contenu d'eau, le coût pour un séchage additionnel atteint un niveau beaucoup trop important, et de plus, le phénomène de pulvérisation (tel que détaillé avec ses problèmes dans le paragraphe d'état de la technique, précité) prend des proportions considérables.

Ces co-inventeurs ont découvert qu'on obtient une performance élevée inattendue, du point de vue des caractéristiques de transfert de chaleur de ce coussin thermique, lorsque le contenu en eau de l'avoine est fixé à l'intérieur d'une fourchette s'étalant de 10 à 11 %. Cette performance inattendue est associée à la hausse marquée des niveaux d'inertie d'humidité, réalisée alors que le contenu d'eau de l'avoine descend, à commencer par une valeur d'environ 14 ou 13.5, jusqu'à une valeur d'inertie d'humidité spectaculairement élevée rendu dans la fourchette de 10 à 11 %. A de telles valeurs élevées d'inertie, même si le niveau d'humidité ambiante de l'air est relativement élevé, le grain d'avoine demeurera extrêmement résistant à un changement (i.e. à une augmentation) de contenu en eau; en effet, cela prendra plusieurs jours pour que le grain d'avoine à son niveau de 10.5 % de contenu en eau puisse augmenter même légèrement, et ce, même dans un environnement très humide.

Bien entendu, le coussin d'avoine devra être entreposé dans un endroit sec, lorsque non utilisé, de façon à ce qu'il puisse maintenir à long terme le contenu faible en eau de ses grains d'avoine.

Au fur et à mesure que le contenu en eau baisse encore plus sous la marque du 10 %, les problèmes apparaissent: les coûts de séchage deviennent progressivement plus élevés, le matériau granulaire se réduit progressivement en poudre ce qui altère la granulométrie bien choisie des grains de céréales, de sorte que les divers problèmes décrits dans le paragraphe précité de l'état de la technique s'accumulent. Ces co-inventeurs ont établi qu'en dessous de 9 % plus ou moins de contenu en eau des grains d'avoine, l'efficacité dans l'exploitation de leur réalisation baissait drastiquement.

Pour le matériau constitutif de cet enveloppe de coussin thermique, le coton est privilégié, parce qu'il ne provoque pas de réaction allergique, parce qu'il est poreux, qu'il est très lisse et donc confortable pour la peau. (C'est pourquoi c'est le matériau préféré pour les couches de bébé). La paroi de l'enveloppe de coton devrait être relativement épaisse - tout en ne l'étant pas trop, autrement on créerait désavantageusement un écran d'eau (les grains d'avoine doivent pouvoir "respirer"). Un exemple d'un coussin thermique fonctionnel selon l'invention comprendrait une enveloppe de coton ayant une densité d'environ 203 g/m² (6 onces par verge carrée), l'enveloppe de coton enfermant une masse d'approximativement un kilo de grains de céréales (préférablement, de l'avoine). L'enveloppe de coton est réalisée à partir d'une feuille de coton unique, pliée en deux et cousue à son bord de périphérie. La ligne de couture est préférablement dirigée vers l'extérieur de ce coussin thermique, de façon à ne nuire d'aucune façon au libre écoulement distributif du matériau granulaire d'avoine, cet écoulement distributif de grains découlant de pliages répétés du coussin thermique autour des parties de membres à être soulagées.

Des matériaux d'enveloppe acceptables autres que le coton pourraient comprendre la flanelle, le GORTEX (une marque de commerce) (un matériau en feuille qui permet la respiration unidirectionnelle, i.e. de l'intérieur vers l'extérieur seulement du coussin), du lin, ou tout autre matériau en tissu, en autant que ce matériau soit du type pouvant aller au four à micro-ondes. Le matériau de l'enveloppe devrait être un matériau qui isolera thermiquement la surface de la peau de la partie de corps par rapport au remplissage en grains de céréale à haute inertie thermique, afin d'éviter une sensation de "brûlure" au moment du contact; des matériaux particulièrement non souhaitables de ce point de vue seraient des surfaces métalliques et semblables ayant de faibles densités thermiques spécifiques.

Les grains d'avoine sont préférés, parce que chaque grain est recouvert d'une surface lisse, généralement ovoïdale, qui sera particulièrement confortable pour la peau, lorsque le coussin rempli de tels grains roulera au-dessus du membre à être soulagé et y sera maintenu. Les grains d'avoine ne dégagent au surplus presque pas d'odeur, lorsqu'ils sont chauffés. Le type de céréales envisagé, autre que l'avoine qui est préféré, comprendra: l'orge, le blé, la farine de sarrasin, le riz, et le maïs. En revanche, par rapport aux grains d'avoine:
(a) le maïs dégage de façon désagréable des odeurs marquées, lorsque chauffé (cependant, il a l'avantage d'être particulièrement léger par rapport aux autres grains de céréales);
(b) les grains de blé ont une surface rude, ou moletée, ce qui n'est pas souhaitable; et
(c) les grains de farine de sarrasin comportent quant à eux des arêtes vives.

Le volume de chargement des grains d'avoine à l'intérieur de l'enveloppe de coton doit être contrôlé de façon précise. Un volume trop grand réduit la flexibilité du coussin. Un volume trop petit entraîne la formation de vides sans grains à l'intérieur du coussin, ce qui réduit donc l'efficacité des propriétés de transfert thermique de ce coussin thermique.

La méthode préférée pour le préchauffage du coussin thermique ayant une charge de un kilo de grains d'avoine dans le coussin thermique (l'enveloppe de coton correspondante à densité de 203 g/m² (6 onces par verge carrée), est "transparente aux radiations dans la gamme des micro-ondes"), s'effectue dans un four à micro-ondes (au degré maximum, pendant une à deux minutes). Cependant, les fours conventionnels à convection ne sont pas exclus; toutefois, l'enveloppe de coton devrait alors être recouverte d'une gaine protectrice, notamment en aluminium, afin d'empêcher la dégradation du coton durant la période de chauffage.

Le coussin thermique pourrait alternativement présenter d'autres formes, y compris la forme d'une pantoufle, d'une chemise ou d'une culotte, ou de tout autre vêtement ou même en fait de tout type de meuble ou autre structure capitonnée. Des chevaux de course pourraient également voir l'articulation de leur pattes soulagée (en cas d'inflammation ou d'enflure) par des coussins de pattes, entourant la partie de la patte, ces coussins de pattes étant remplis de grains d'avoine (ou d'autre céréale) selon la présente invention, ces coussins d'avoine étant pré-congelés à l'intérieur d'un congélateur pour un certain nombre d'heures. Le coussin pourrait également suggérer la forme d'une pochette pour recevoir une bouteille de vin, pour refroidir le vin blanc avant le service. Ou, par exemple, encore une autre variante serait de prévoir un coussin de coton divisé en un certain nombre de pochettes séparées ou de "cellules", chaque cellule étant séparée des cellules adjacentes par des lignes de couture; chaque cellule contenant un remplissage de grains de céréales qui ne soient pas en contact avec les grains des autres cellules; un tel coussin serait particulièrement avantageux pour des portions du corps qui sont coudées, e.g. la région des épaules, de sorte que le coussin puisse intimement épouser les contours du corps.

Dans l'une quelconque de ces variantes, les caractéristiques de transfert thermique demeurent les mêmes, à savoir:
- lorsque les grains d'avoine sont congelés, cela permet d'obtenir le soulagement des bursites, des migraines, des entorses, de la fièvre, et de la tendinite; et
- lorsque les grains d'avoine sont chauffés, cela permet d'obtenir le soulagement des douleurs musculaires, des dérangements d'estomac, de l'arthrite, de l'arthrose, du torticolis, de la tendinite, de la bursite, ou pour simplement relaxer.

Ce coussin thermique doit demeurer sec en tout temps (il perdra ses caractéristiques avantageuses s'il est immergé dans un volume d'eau pour un certain temps).

Il est entendu que les grains de céréales possèdent un contenu en eau qui est naturellement assez bas.

## Revendications

1. Un coussin thermothérapeutique destiné à être utilisé pour le soulagement des membres du corps d'un mammifère, comprenant une enveloppe, réalisée en un matériau en feuille flexible, lisse, poreux, conducteur thermique, constitué par le coton, ladite feuille en coton définissant une ligne périphérique de couture délimitant l'enceinte formée par ladite enveloppe, et un matériau granulaire sans aucun liquide libre, ledit matériau granulaire étant enfermé dans ladite enveloppe et étant retenu par celle-ci;
caractérisé en ce que ledit matériau granulaire consiste en des grains de céréales, lesdits grains de céréales étant des grains d'avoine et étant non saturés et non liés les uns aux autres et ayant un contenu en eau variant entre 10 et 11 % par poids des grains de céréales, de sorte qu'on obtienne un écoulement libre bien réparti de ceux-ci à l'intérieur de ladite enveloppe lorsque le coussin est plié de façon à épouser les contours d'un membre du corps, lesdits grains de céréale étant caractérisés par une inertie thermique élevée de même que par une inertie d'humidité élevée.

2. Un coussin thermothérapeutique tel que défini à la revendication 1,
caractérisé en ce que ladite ligne de couture de la feuille en coton est orientée vers l'extérieur par rapport à ladite enceinte de l'enveloppe, de sorte que ladite enceinte de l'enveloppe définit une surface intérieure lisse telle qu'on favorise un libre écoulement équi-réparti des grains à l'intérieur du coussin, lorsque le coussin est plié.

3. Un coussin thermothérapeutique tel que défini à la revendication 2,
caractérisé en ce que lesdits grains d'avoine définissent une granulométrie sensiblement constante, ladite granulométrie de grain étant spécifiquement choisie pour permettre un écoulement libre bien réparti des grains à l'intérieur de ladite enveloppe de coussin, même après de nombreux pliages du coussin, tout en empêchant clairement la formation de vides sans grains dans l'enceinte formée à l'intérieur de ladite enveloppe du coussin, aux portions coudées du coussin thermique plié; ladite granulométrie du grain demeurant sensiblement constante durant l'emploi du coussin, même après son pliage répété.

4. Un coussin thermothérapeutique tel que défini à la revendication 1,
caractérisé en ce que la densité de ladite enveloppe de coton est approximativement de 203 g\m² (six onces par verge carrée).

## Claims

1. A thermotherapeutic pad for use in soothing mammal body limbs, comprising an envelope, made from a flexible, smooth, porous, thermally conductive sheet material, consisting of cotton, said cotton sheet defining a peripheral stitch line circumscribing the enclosure defined by said envelope, and a granular material exclusively of any free liquid , said granular material being enclosed within and retained by said envelope; wherein said granular material consists of oat grains of cereals, said oat grains of cereal being unsaturated and unbound to one another and having a water content set to range between 10 and 11 % by weight of the cereal grains, wherein free distributive flow thereof within said envelope during pad flexing conformingly against a body limb is enabled, said grains of cereal being characterized by a high moisture inertia as well as by a high thermal inertia.

2. A thermotherapeutic pad as defined in claim 1,
wherein said cotton sheet stitch line is oriented outwardly from said envelope enclosure, wherein said envelope enclosure defines a smooth inner surface whereby free distributive grain flow within the pad envelope is promoted during pad flexing.

3. A thermotherapeutic pad as defined in claim 2,
wherein said oat grains define a substantially constant, set granulometry, said grain granulometry being specifically selected to enable free distributive flow of grains within said pad envelope even after repeated pad flexing, while positively preventing the formation of grainless pockets inside the pad envelope enclosure, at the elbowed portions of the flexed thermal pad; said grain granulometry remaining substantially constant throughout use, even after repeated pad flexings.

4. A thermotherapeutic pad as defined in claim 1,
wherein the density of said cotton envelope is approximately of 203 g\m² (six ounces per square yard).

## Patentansprüche

1. Thermotherapeutisches Kissen, das dazu bestimmt ist, zur Linderung bei Körpergliedern eines Säugetiers verwendet zu werden, das eine Hülle, die aus einem flexiblen, glatten, porösen, wärmeleitfähigen, aus Baumwolle bestehenden Lagenmaterial ausgeführt ist, wobei die Baumwolllage eine Umfangsnahtlinie definiert, die den von der Hülle gebildeten Raum begrenzt, und ein körniges Material ohne jegliche freie Flüssigkeit umfaßt, wobei das körnige Material in der Hülle eingeschlossen ist und von dieser zurückgehalten wird,
dadurch gekennzeichnet, daß das körnige Material aus Getreidekörnern besteht, wobei die Getreidekörner Haferkörner sind und nicht gesättigt und nicht miteinander verbunden sind und einen Wassergehalt haben, der zwischen 10 und 11 Gew.-% der Getreidekörner variiert, so daß ein freies, gut verteiltes Fließen dieser im Inneren der Hülle erzielt wird, wenn das Kissen so gefaltet wird, daß es sich den Konturen eines Körperglieds anpaßt, wobei die Getreidekörner durch einen erhöhten Wärmeleitwiderstand sowie durch einen erhöhten Feuchtigkeitswiderstand gekennzeichnet sind.

2. Thermotherapeutisches Kissen nach Anspruch 1,
dadurch gekennzeichnet, daß die Nahtlinie der Baumwolllage in bezug auf den Raum der Hülle nach außen gerichtet ist, so daß der Raum der Hülle eine glatte Innenfläche definiert, so daß ein freies, gleichverteiltes Fließen der Körner im Inneren des Kissens gefördert wird, wenn das Kissen gefaltet wird.

3. Thermotherapeutisches Kissen nach Anspruch 2,
dadurch gekennzeichnet, daß die Haferkörner eine im wesentlichen konstante Granulatgeometrie definieren, wobei die Korngranulatgeometrie entsprechend gewählt ist, um selbst nach zahlreichen Faltungen des Kissens ein gut verteiltes, freies Fließen der Körner im Inneren der Kissenhülle unter deutlicher Verhinderung der Bildung von leeren Räumen ohne Körner im vom Inneren der Hülle des Kissens gebildeten Raum in abgewinkelten Teilen des gefalteten, thermischen Kissens zu gestatten; wobei die Korngranulatgeometrie während des Einsatzes des Kissens selbst nach wiederholten Faltungen im wesentlichen konstant bleibt.

4. Thermotherapeutisches Kissen nach Anspruch 1,
dadurch gekennzeichnet, daß die Dichte der Baumwollhülle ungefähr 203 g/m² (sechs Unzen pro Quadratyard) beträgt.
